# FASCICULE DE BREVET EUROPEEN

(11) **EP 1 490 265 B1**
(45) Date de publication et mention de la délivrance du brevet: **21.06.2006**
(21) Numéro de dépôt: 03740538.8
(22) Date de dépôt: 28.03.2003
(51) Int. Cl.: B65B 55/08, B65B 55/10

(54) **PROCEDE ET INSTALLATION POUR LA DECONTAMINATION DES COLS DE PREFORMES**
VERFAHREN UND VORRICHTUNG ZUR DEKONTAMINATION VON VORFORMLINGHÄLSEN
METHOD AND INSTALLATION FOR DECONTAMINATING PREFORM NECKS

(30) Priorité: 04.04.2002 FR 0204202
(43) Date de publication de la demande: 29.12.2004
(73) Titulaire: SIDEL, 76930 Octeville-sur-Mer (FR)
(72) Inventeur: QUETEL, François, F-76930 Octeville sur Mer (FR); MIE, Patrick, F-76930 Octeville sur Mer (FR)
(74) Mandataire: Gorrée, Jean-Michel
(86) Numéro de dépôt international: PCT/FR2003/000986
(87) Numéro de publication internationale: WO 2003/084818

(56) Documents cités:
- EP-A- 0 342 690
- WO-A-95/11765
- US-A- 4 375 145
- US-A- 5 129 212
- US-A- 6 145 276
- US-B1- 6 183 691

## Description

La présente invention se situe, d'une façon générale, dans le domaine de la décontamination (ou abaissement du taux de contamination) des préformes en matière thermoplastique (notamment en PET) destinées à la fabrication de récipients, notamment de bouteilles, flacons, ..., par un processus de soufflage ou d'étirage-soufflage, et elle concerne plus spécifiquement des perfectionnements apportés dans l'étape de décontamination des cols de ces préformes.

Pour la fabrication de récipients décontaminés en matière thermoplastique, il est connu de décontaminer les préformes, en début du processus de fabrication des récipients, plutôt que les récipients achevés, en fin de leur processus de fabrication, afin de réduire notablement la quantité de produit de décontamination employée (cette quantité étant fonction de la surface à traiter).

A titre d'exemple, on connaît, d'après le document FR-A-2 766 121, un procédé et une installation de décontamination de préformes qui consistent à mouiller les corps des préformes en les trempant dans un bain d'un produit de décontamination tel que le peroxyde d'hydrogène, puis à activer thermiquement ce produit de décontamination par chauffage de la préforme (l'activation du produit de décontamination peut intervenir, notamment, dans le four de chauffage des préformes en vue de l'étape de soufflage).

Toutefois une décontamination efficace des préformes nécessite que la totalité de chaque préforme soit décontaminée, c'est-à-dire non seulement son corps, mais aussi son col. Or il est connu que les préformes sont fabriquées par moulage avec leur col conformé et dimensionné de façon définitive et il est indispensable que les cols ne soient soumis ensuite à aucune contrainte thermique qui, sinon, engendrerait des déformations des cols et risquerait de rendre impossible le bouchage ultérieur des récipients remplis.

C'est la raison pour laquelle le procédé de décontamination avec activation thermique exposé dans le document précité ne peut s'appliquer qu'aux corps des préformes et c'est également la raison pour laquelle, pour décontaminer les cols, on met en oeuvre un autre processus non thermique qui, classiquement, est une décontamination par ultraviolets, qui consiste à placer chaque col de préforme sous un rayonnement ultraviolet pendant un laps de temps prédéterminé. A cet effet, dans une mise en oeuvre concrète, des lampes à rayonnement ultraviolet sont réparties de part et d'autre d'une glissière inclinée sur laquelle les préformes glissent par gravité depuis une trémie d'alimentation jusqu'à un dispositif de préhension et de chargement dans l'installation, la durée d'exposition des cols au rayonnement ultraviolet étant déterminée par la vitesse de descente des préformes sur la glissière et la longueur de cette dernière.

Toutefois, le niveau de décontamination ainsi obtenu est relativement faible (par exemple de l'ordre de 1 à 1,5 log, c'est-à-dire une réduction par 10 à 30), bien qu'il puisse être suffisant pour certaines applications.

Par contre, pour d'autres applications qui requièrent un niveau de décontamination plus élevé (par exemple d'au moins 3 log, c'est-à-dire une réduction par 1000), le processus de décontamination par rayonnement ultraviolet est insuffisant.

Le document EP 0 342 690 concerne une installation permettant, notamment, la stérilisation au défilé de récipients en forme de pots, installation dans laquelle du liquide de stérilisation atomisé est projeté sur les récipients. Toutefois, dans cette installation connue, le liquide atomisé projeté n'est pas canalisé, de sorte qu'il se disperse au sein de l'installation et que chaque récipient n'est mouillé que par la fraction de jet qu'il intercepte. Dans le cas où il s'agirait non pas de récipients en forme de pot largement ouvert, mais de récipients à col étroit tels que des bouteilles, on ne serait pas assuré d'un mouillage homogène et suffisant à la fois de la face externe et de la face interne des cols. En outre, une telle installation exige une quantité importante de liquide de stérilisation, ce qui se révèle coûteux.

Le document US 5 129 212 concerne une installation de stérilisation de récipients équipée d'une chambre qui sert à la fois comme lieu d'injection d'un produit de stérilisation et comme lieu d'application d'un rayonnement ultraviolet. Cette installation connue présente l'inconvénient majeur que, dès sa projection, le liquide de stérilisation est soumis à l'action du rayonnement ultraviolet, et c'est donc un produit déjà en partie dénaturé qui atteint les récipients. Il est donc nécessaire, pour obtenir une stérilisation efficace, de dispenser une quantité accrue de liquide, ce qui se révèle dispendieux.

L'invention a donc pour but de perfectionner le processus de décontamination des cols de préformes de manière qu'il soit possible d'atteindre un niveau de décontamination supérieur à celui conféré par le seul traitement aux ultraviolets, avec en outre l'exigence supplémentaire de ne pas accroître notablement la complexité et surtout l'encombrement de l'ensemble de l'installation de fabrication des récipients.

A cet effet, selon un premier de ses aspects, l'invention propose un procédé pour décontaminer le col de préformes en matière thermoplastique destinées à la fabrication de récipients par un processus de soufflage ou d'étirage-soufflage,
caractérisé en ce que, lors de l'alimentation des préformes les unes à la suite des autres dans une unité de fabrication des récipients, les préformes traversent d'abord une chambre située en amont dans laquelle un liquide de décontamination est pulvérisé en permanence de manière à y entretenir une atmosphère de brouillard dudit produit de décontamination au contact duquel sont mis les cols des préformes, puis passent en regard de lampes à rayonnement ultraviolet disposées de manière à irradier, en totalité, les cols des préformes mouillés par le produit de décontamination pendant au moins une durée minimale prédéterminée, avant de parvenir à un dispositif de chargement dans l'unité de fabrication.

De façon préférée, pour assurer l'efficacité optimale du procédé, on entretient une circulation du brouillard pour faciliter le renouvellement de celui-ci.

Dans un mode de mise en oeuvre pratique, le produit de décontamination est du peroxyde d'hydrogène H₂O₂, ce produit étant connu pour son efficacité et son coût relativement modique.

Grâce aux dispositions de l'invention, le fait que les cols de préformes traversent un brouillard de produit de décontamination conduit à un mouillage des cols extérieurement, sur le buvant (rebord), et en partie intérieurement, sans qu'il soit nécessaire de réaliser une projection du liquide sous forme de jets ou sans qu'il soit nécessaire d'immerger les cols dans un bain de liquide de décontamination comme cela se pratique pour le traitement des corps. Il en résulte un mouillage tout aussi efficace des cols avec l'utilisation d'une quantité moindre de liquide et avec une moindre complication en matériels.

En outre, cette formation de brouillard se situe en amont des rampes de lampes à rayonnement ultraviolet qui demeurent en place le long de la trajectoire suivie par les cols des préformes, de sorte que les modifications structurelles à apporter à l'installation sont minimes et surtout parfaitement localisées en tête des moyens de déplacement des préformes.

Enfin, sous l'action du rayonnement ultraviolet, le liquide de décontamination déposé sur les cols des préformes est activé et le processus de décontamination s'accomplit sans qu'il en résulte une atteinte quelconque à la conformation des cols.

A titre d'exemple, utilisant une solution de peroxyde d'hydrogène à 1 %, la durée d'exposition des cols au rayonnement ultraviolet doit être typiquement d'au moins 8 secondes, ce qui implique que la longueur et la vitesse du déplacement des préformes (cette dernière étant elle-même fonction de la vitesse de fonctionnement de l'ensemble de l'installation) soient déterminées en conséquence.

Selon un second de ses aspects, pour la mise en oeuvre du procédé ci-dessus, l'invention propose une installation pour décontaminer au défilé les cols de préformes délivrées les unes à la suite des autres à un dispositif de chargement, lesdites préformes étant constituées en matière thermoplastique et étant destinées à la fabrication de récipients par soufflage ou étirage-soufflage, ladite installation de décontamination étant structurellement et fonctionnellement associée avec une installation d'alimentation des préformes comprenant des moyens de déplacement des préformes les unes à la suite des autres,
ladite installation de décontamination comprenant des lampes à rayonnement ultraviolet disposées de manière que le rayonnement ultraviolet irradie totalement les cols des préformes en déplacement, caractérisée en ce que l'installation de décontamination comprend en outre, disposée en amont des lampes à rayonnement ultraviolet, une chambre traversée par lesdits moyens de déplacement des préformes de l'installation d'alimentation et dans laquelle sont prévus des moyens de pulvérisation d'un produit de décontamination propres à entretenir un brouillard du produit de décontamination dans ladite chambre.

Avantageusement, les moyens de pulvérisation comprennent au moins deux buses de pulvérisation disposées de part et d'autre des moyens de déplacement des préformes et au-dessus de ceux-ci, avec leurs axes respectifs dirigés sensiblement en direction des cols des préformes en déplacement.

Pour obtenir un fonctionnement efficace, on prévoit en outre que des moyens d'aspiration soient raccordés à la chambre de manière à créer dans celle-ci un courant de circulation propre à empêcher des accumulations locales de produit en suspension.

Selon les moyens de déplacement utilisés à l'intérieur de la chambre, il peut être nécessaire de les compléter avec un organe anti-soulèvement des préformes qui permet par ailleurs de laisser l'accès au brouillard de produit décontaminant à la paroi intérieure des cols des préformes : dans un mode de réalisation préféré, cet organe peut être une tige, surmontant les cols des préformes, de relativement faible dimension transversale par rapport au diamètre des cols.

Ainsi, l'installation conforme à l'invention se distingue par la présence de la chambre à brouillard en tête des moyens de déplacement des préformes, tandis qu'en aval l'équipement des moyens de déplacement des préformes avec des rampes de lampes à rayonnement ultraviolet demeure inchangé. Il en résulte un aménagement très localisé de l'installation d'alimentation en préformes qui n'entraîne des modifications ni dans la partie amont (trémie et présentation des préformes une par une), ni dans la partie aval (traitement par rayonnement ultraviolet et déchargement dans l'installation).

Dans un mode de réalisation préféré qui correspond à un agencement de ce type d'installation, les moyens de déplacement des préformes comprennent une glissière inclinée sur laquelle les préformes glissent par gravité les unes à la suite des autres et en ce que la chambre est traversée par cette glissière.

L'invention sera mieux comprise à la lecture de la description détaillée qui suit d'un mode de réalisation préféré donné uniquement à titre d'exemple non limitatif. Dans cette description, on se réfère aux dessins annexés sur lesquels :
- la figure 1 est une vue schématique de côté de l'ensemble d'une installation de décontamination de cols de préformes combinée avec une installation d'alimentation de préformes, conformément à l'invention ;
- la figure 2 est une vue de côté d'une partie de l'installation de la figure 1 ; et
- la figure 3 est une vue en coupe transversale de la chambre à brouillard incluse dans la partie de l'installation visible à la figure 2.

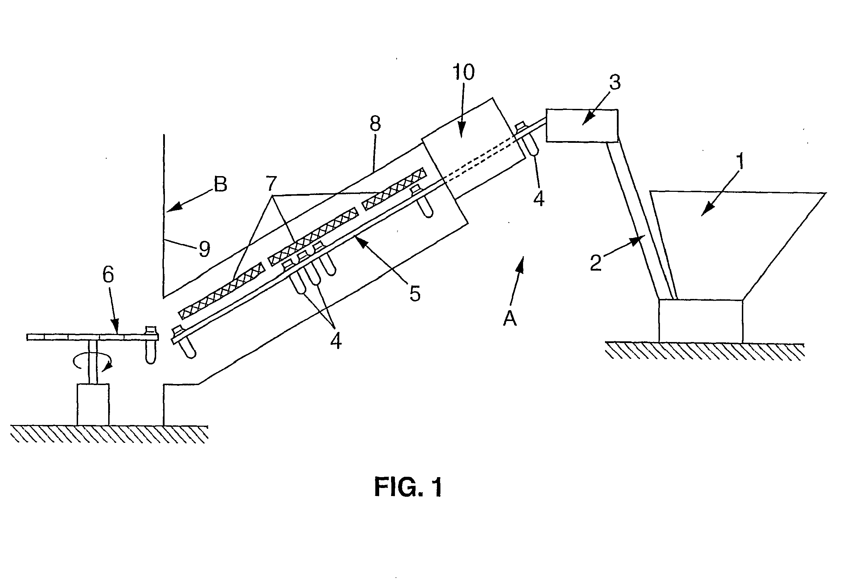

A la figure 1 est illustrée schématiquement une installation A d'alimentation en préformes qui constitue l'unité d'entrée d'une installation B de fabrication de récipients en matière thermoplastique par soufflage ou étirage-soufflage.

Un agencement classique d'installation d'alimentation comprend principalement une trémie 1 dans laquelle sont déversées en vrac les préformes en matière thermoplastique tel que le PET, trémie à la base de laquelle les préformes sont prélevées une par une par un dispositif ascenseur 2 qui les amène en hauteur à un dispositif 3 de présentation en position correcte, les une à la suite des autres en position verticale avec le col en haut.

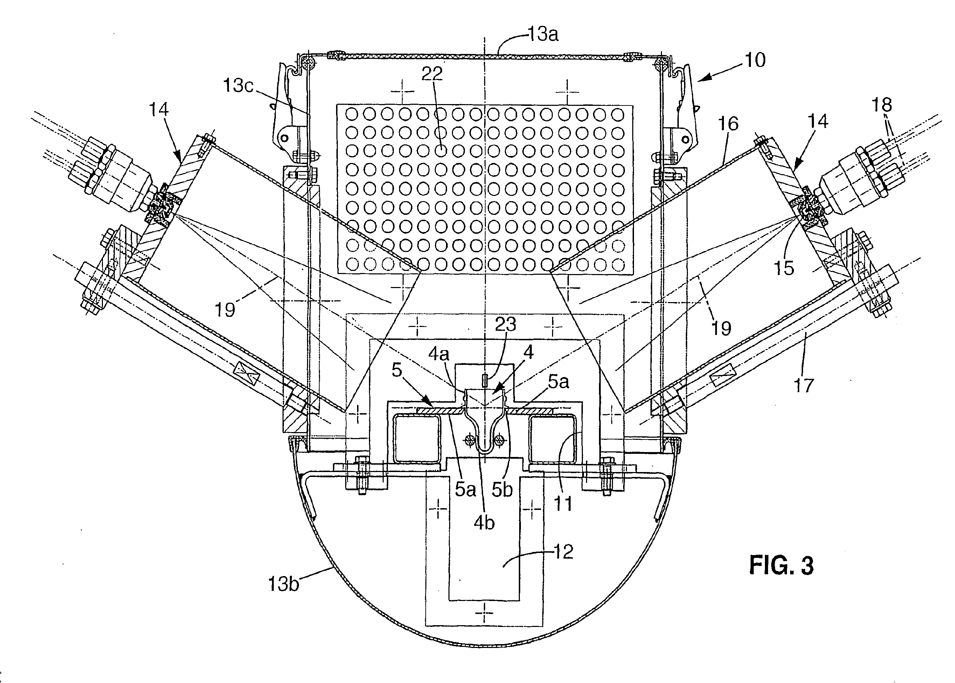

A la sortie du dispositif 3 de présentation, les préformes 4 passent sur une glissière 5 inclinée sur laquelle elles se déplacent par gravité, les unes à la suite des autres. De façon classique, la glissière 5 est constituée de deux rails parallèles écartés l'un de l'autre. Les préformes reposent, par une collerette externe située à la base de leur col, à cheval sur le sommet des deux rails, tandis que leur corps est engagé et guidé dans l'intervalle entre les rails (voir la vue à plus grande échelle de la figure 3).

A l'extrémité inférieure de la glissière 5, les préformes 4, qui sont présentées une à une, sont saisies individuellement par un dispositif préhenseur en général constitué sous forme d'une roue de chargement 6. La roue de chargement 6 constitue en pratique l'organe d'entrée de l'installation B de fabrication de récipients et, typiquement, elle délivre les préformes à l'entrée d'un four de chauffage des préformes (non montré).

S'agissant, dans le cadre de l'installation plus spécifiquement visée par l'invention, d'une installation de décontamination, la roue de chargement 6 délivre les préformes à une unité de décontamination des corps de préformes (non montrée) qui peut, notamment, être combinée avec le four de chauffage des préformes comme indiqué plus haut.

Pour la décontamination des seuls cols des préformes - lesquels ne doivent être soumis à aucun traitement thermique -, on dispose des rampes de lampes 7 à rayonnement ultraviolet en association avec la glissière 5. Ces rampes de lampes 7 sont disposées sur les côtés de part et d'autre de la glissière et au-dessus de la glissière de manière que les cols des préformes soient irradiés en totalité (extérieurement, intérieurement et sur le buvant) par le rayonnement ultraviolet. Pour protéger les préformes ainsi traitées, la glissière 5 et les rampes de lampes 7 à rayonnement ultraviolet sont enfermées dans un capotage 8 en forme de tunnel qui se raccorde à celui 9 de l'installation B, l'ensemble pouvant avantageusement être mis en surpression.

La décontamination des cols de préformes par la seule action du rayonnement ultraviolet conduisant seulement à un niveau limité de décontamination (typiquement 1 à 1,5 log), une décontamination d'un niveau accru (par exemple typiquement 3 log) ne peut être obtenue qu'en soumettant les cols de préformes à l'action d'un produit décontaminant tel que le peroxyde d'hydrogène. Pour éviter d'avoir à tremper les cols des préformes dans un bain de produit ou à projeter des jets de liquide sur les cols des préformes - processus qui conduiraient à des installations complexes, encombrantes et coûteuses -, l'invention prévoit la mise en oeuvre d'un brouillard de produit décontaminant à travers lequel défilent les cols des préformes ; une fois mouillés, les cols passent sous le rayonnement ultraviolet qui active le produit décontaminant et conduit au niveau requis de décontamination des cols.

Pour ce faire, on prévoit de compléter l'installation décrite ci-dessus à l'aide des moyens suivants.

Entre le dispositif 3 de présentation correcte des préformes à l'entrée supérieure de la glissière 5 et les rampes de lampes 7 à rayonnement ultraviolet, et donc en amont desdites rampes de lampes 7, on dispose une chambre 10 fermée de manière toutefois à être traversée par la glissière 5 et à laisser un libre passage aux préformes 4. Dans la chambre 10 on entretient un brouillard de produit de décontamination tel que le peroxyde d'hydrogène de façon à humidifier les cols des préformes 4 sur leur face extérieure, sur leur buvant et en partie sur leur face intérieure.

Grâce à cet agencement, les préformes 4, au fur et à mesure qu'elles descendent sur la glissière 5, traversent la chambre 10 à brouillard où leur col est mouillé par le produit décontaminant, puis, en aval de cette chambre 10, passent entre les rampes de lampes 7 à rayonnement ultraviolet où le rayonnement ultraviolet active le produit décontaminant. La durée du parcours des cols de préformes sous le rayonnement ultraviolet est déterminée pour être suffisamment longue pour conduire à une décontamination efficace des cols des préformes (par exemple durée de l'ordre de 8 secondes avec du peroxyde d'hydrogène à 1 % à température ambiante).

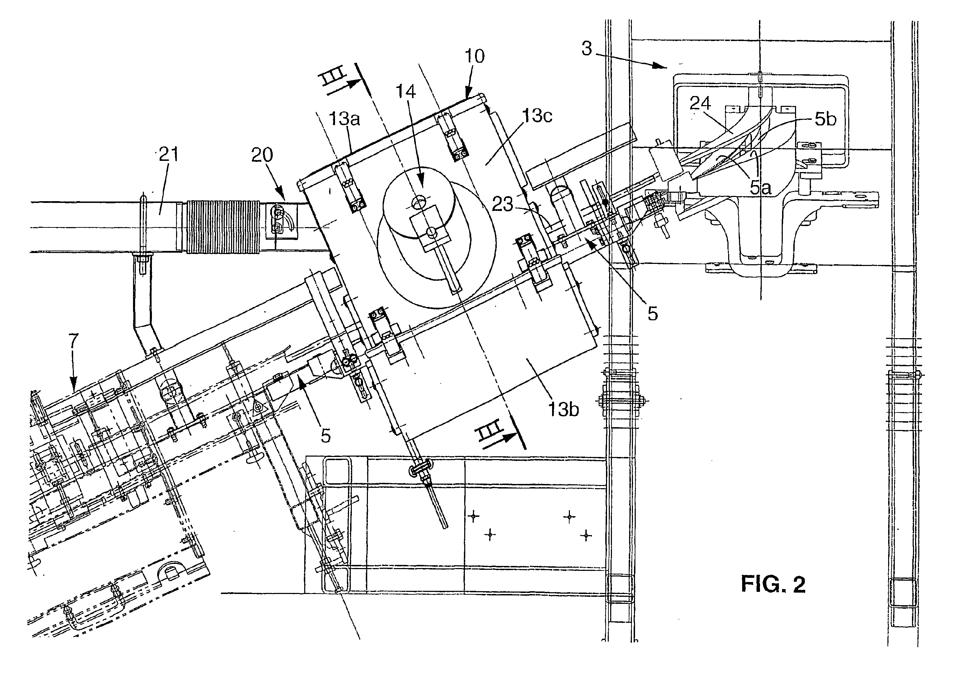

L'agencement spécifique de la chambre 10 est illustré de façon détaillée et à plus grande échelle à la figure 2, qui est une vue de côté analogue à celle de la figure 1, et à la figure 3, qui est une vue en coupe transversale selon la ligne III-III de la figure 2. Sur ces figures 2 et 3, on a repris les mêmes références numériques qu'à la figure 1 pour désigner les mêmes organes ou parties fonctionnelles. Sur la figure 2, l'habillage protecteur 8 a été enlevé pour rendre la figure mieux lisible. A la partie supérieure de la glissière 5 qui est en courbe dans le mode de réalisation représenté, on remarque les deux rails 5a sur lesquels les préformes 4 visibles sur la figure 3 reposent par leur collerette, avec leur col 4a surmontant la glissière 5 et leur corps 4b engagé dans l'intervalle 5b défini entre les deux rails 5a.

La chambre 10 est constituée par exemple en tôle et entoure, à sa partie inférieure, la glissière 5. Ses deux faces de bout sont découpées pour présenter deux ouvertures 11 respectives juste conformées pour donner libre passage aux préformes. Eventuellement des caches amovibles peuvent être prévus pour obturer des portions de ces ouvertures 11 lorsque les préformes sont de petite dimension (par exemple cache 12 prévu dans le cas, illustré à la figure 3, de préformes à corps courts).

La chambre 10 peut être constituée par assemblage de plusieurs éléments amovibles (couvercle 13a, fond 13b) solidarisés à une structure principale 13c, aux fins de faciliter l'entretien.

Des moyens 14 de pulvérisation fine du produit décontaminant sont associés à la chambre 10 de manière que soit entretenu dans celle-ci un brouillard de produit décontaminant. Ces moyens 14 peuvent, de préférence, être doubles et être disposés de part et d'autre de la glissière 5 comme illustré à la figure 3. Chacun de ces moyens pulvérisateurs comprend une buse 15 de pulvérisation fine montée sur la paroi de fond d'un carter 16 cylindrique engagé à travers une ouverture pratiquée dans la paroi de la structure principale 13c de la chambre 10 et fixé à celle-ci par des moyens de fixation 17. La buse 15 est raccordée à une source de produit décontaminant sous pression par un ou des conduits 18.

Les deux moyens pulvérisateurs 14 sont disposés de part et d'autre et au-dessus de la glissière 5, de façon sensiblement symétrique, avec les axes 19 respectifs des buses 15 qui sont sensiblement coplanaires et qui sont dirigés sensiblement sur les cols 4a des préformes 4.

Grâce à ces dispositions, on constitue un agencement structurellement simple, dont les pièces ou parties composantes sont faciles à démonter et/ou sont d'accès facile. Intérieurement, la chambre 10 ne présente que peu de reliefs et peu de recoins (en particulier, le fond 13b peut être conformé en demi-cylindre) de sorte qu'on réduit les possibilités de dépôt de produit ou de confinement du brouillard.

Pour encore améliorer l'efficacité du renouvellement du brouillard et d'un mouillage rapide et homogène des cols 4a des préformes, on prévoit de pourvoir la chambre 10 d'un dispositif d'aspiration 20 (voir figure 2) constitué par exemple par une ouverture pratiquée dans la paroi de la structure principale 13c de la chambre, à laquelle est raccordée un conduit 21 relié par exemple à un aspirateur (non visible). Simultanément, une des parois de la chambre peut être perforée (comme illustré en 22 à la figure 3) pour favoriser une circulation à l'intérieur de la chambre.

On notera également que, pour ne pas gêner le passage du brouillard de produit décontaminant vers la paroi intérieure des cols des préformes, on prévoit, au-dessus de la glissière et au-dessus des cols 4a des préformes, un organe anti-soulèvement (s'opposant au soulèvement accidentel d'une préforme hors de la glissière 5) sous forme d'une tige 23 de section transversale relativement faible par rapport au diamètre des cols 4a - et non pas sous forme d'une bande plate relativement large comme c'est le cas sur le reste de la glissière (voir par exemple la bande 24 en amont de la chambre 10 à la figure 2).

Bien entendu, la longueur de la chambre 10, aussi bien que le nombre et la disposition des moyens pulvérisateurs 14 peuvent être adaptés en fonction des conditions générales de fonctionnement de l'ensemble de l'installation de fabrication des récipients, et notamment en fonction de la vitesse de déplacement des préformes sur la glissière 5.

## Revendications

1. Procédé pour décontaminer le col de préformes en matière thermoplastique destinées à la fabrication de récipients par un processus de soufflage ou d'étirage-soufflage,
**caractérisé en ce que**, lors de l'alimentation des préformes les unes à la suite des autres dans une unité de fabrication des récipients, les préformes traversent d'abord une chambre située en amont dans laquelle un liquide de décontamination est pulvérisé en permanence de manière à y entretenir une atmosphère de brouillard dudit produit de décontamination au contact duquel sont mis les cols des préformes, puis passent en regard de lampes à rayonnement ultraviolet disposées de manière à irradier, en totalité, les cols des préformes mouillés par le produit de décontamination pendant au moins une durée minimale prédéterminée, avant de parvenir à un dispositif de chargement dans l'unité de fabrication.

2. Procédé selon la revendication 1, **caractérisé en ce qu'**on entretient une circulation du brouillard pour faciliter le renouvellement de celui-ci.

3. Procédé selon la revendication 1 ou 2, **caractérisé en ce que** le produit de décontamination est du peroxyde d'hydrogène H₂O₂.

4. Installation pour décontaminer au défilé les cols (4a) de préformes (4) délivrées les unes à la suite des autres à un dispositif de chargement (6), lesdites préformes (4) étant constituées en matière thermoplastique et étant destinées à la fabrication de récipients par soufflage ou étirage-soufflage, ladite installation de décontamination étant structurellement et fonctionnellement associée avec une installation (A) d'alimentation des préformes comprenant des moyens de déplacement des préformes (4) les unes à la suite des autres,
ladite installation de décontamination comprenant des lampes (7) à rayonnement ultraviolet disposées de manière que le rayonnement ultraviolet irradie totalement les cols (4a) des préformes (4) en déplacement,
**caractérisée en ce que** l'installation de décontamination comprend en outre, disposée en amont des lampes (7) à rayonnement ultraviolet, une chambre (10) traversée par lesdits moyens de déplacement des préformes de l'installation (A) d'alimentation et dans laquelle sont prévus des moyens (14) de pulvérisation d'un produit de décontamination propres à entretenir un brouillard du produit de décontamination dans ladite chambre.

5. Installation selon la revendication 4, **caractérisée en ce que** les moyens (14) de pulvérisation comprennent au moins deux buses (15) de pulvérisation disposées de part et d'autre des moyens de déplacement des préformes et au-dessus de ceux-ci, avec leurs axes (19) respectifs dirigés sensiblement en direction des cols (4a) des préformes (4) en déplacement.

6. Installation selon la revendication 4 ou 5, **caractérisée en ce que** des moyens (20) d'aspiration sont raccordés à la chambre (10) de manière à créer dans celle-ci un courant de circulation propre à empêcher des accumulations locales de produit en suspension.

7. Installation selon l'une quelconque des revendications 4 à 6, **caractérisée en ce qu'**à l'intérieur de la chambre (10), les moyens de déplacement des préformes sont surmontés, au-dessus des cols (4a) des préformes, d'une tige (23) de relativement faible dimension transversale par rapport au diamètre des cols, cette tige constituant un organe empêchant le soulèvement des préformes tout en laissant accès au brouillard de produit décontaminant à la paroi intérieure des cols des préformes.

8. Installation selon l'une quelconque des revendications 4 à 7, **caractérisée en ce que** les moyens de déplacement des préformes comprennent une glissière (5) inclinée sur laquelle les préformes (4) glissent par gravité les unes à la suite des autres et **en ce que** la chambre (10) est traversée par cette glissière (5).

## Claims

1. A method for decontaminating the necks of thermoplastic preforms intended for making into containers by a blow molding or stretch-blow molding process,
**characterized in that**, as the preforms are fed one after the other into a container manufacturing unit, the preforms pass first through an upstream chamber into which a decontaminating liquid is sprayed continuously so as to maintain in this chamber a fog atmosphere of said decontaminating product with which the necks of the preforms are brought into contact, and then pass in front of ultraviolet lamps arranged so as to completely irradiate the necks of the preforms wetted by the decontaminating product for at least a minimum predetermined period of time, before reaching a device that loads them into the manufacturing unit.

2. The method as claimed in claim 1, **characterized in that** the fog is kept flowing through so as to facilitate its renewal.

3. The method as claimed in claim 1 or 2, **characterized in that** the decontaminating product is hydrogen peroxide H₂O₂.

4. An installation for the decontamination while they are moving of the necks (4a) of preforms (4) delivered one after the other to a loading device (6), said preforms (4) being made of thermoplastic and being intended for making into containers by blow molding or stretch-blow molding, said decontamination installation being structurally and functionally associated to a preform feeder installation (A) comprising means for moving the preforms (4) one after the other,
said decontamination installation comprising ultraviolet lamps (7) arranged so that the ultraviolet radiation completely irradiates the necks (4a) of the moving preforms (4),
**characterized in that** the decontamination installation also includes, upstream of the ultraviolet lamps (7), a chamber (10) traversed by said preform movement means of the feeder installation (A) and in which means (14) are provided for spraying a decontaminating product in such a way as to maintain a fog of the decontaminating product inside said chamber.

5. The installation as claimed in claim 4, **characterized in that** the spray means (14) comprise at least two spray nozzles (15) arranged one on either side of the preform movement means and above these, with their respective axes (19) substantially directed in the direction of the necks (4a) of the moving preforms (4).

6. The installation as claimed in claim 4 or 5, **characterized in that** suction means (20) are connected to the chamber (10) in order to create a flow through the latter such as to prevent local accumulations of product in suspension.

7. The installation as claimed in any one of claims 4 to 6, **characterized in that** inside the chamber (10), the preform movement means are surmounted, above the necks (4a) of the preforms, by a rod (23) of relatively small transverse dimension relative to the diameter of the necks, this rod forming a member that prevents the preforms being lifted up but allows access by the fog of decontaminating product to the inside wall of the necks of the preforms.

8. The installation as claimed in any one of claims 4 to 7, **characterized in that** the preform movement means comprise an inclined slideway (5) down which the preforms (4) slide by gravity one after the other and **in that** this slideway (5) passes through the chamber (10).

## Patentansprüche

1. Verfahren zum Dekontaminieren des Halses von Vorformen aus thermoplastischem Material, die für die Herstellung von Behältern mit einem Blas- oder Blas/Zieh-Verfahren bestimmt sind, **dadurch gekennzeichnet, dass** die Vorformen bei der Zuführung der Vorformen nacheinander in eine Herstellungseinheit für die Behälter zuerst eine Kammer durchqueren, die sich stromaufwärts befindet, in der eine Dekontaminierungsflüssigkeit kontinuierlich zerstäubt wird, um hier eine Nebelatmosphäre des Dekontaminierungsproduktes aufrechtzuerhalten, mit der die Hälse der Vorformen in Kontakt gebracht werden, dann an UV-Lampen vorbeilaufen, die derart angeordnet sind, dass sie die mit dem Dekontaminierungsprodukt befeuchteten Hälse der Vorformen zumindest während einer vorbestimmten Mindestdauer zur Gänze bestrahlen, bevor sie zu einer Beschickungsvorrichtung in einer Herstellungseinheit gelangen.

2. Verfahren nach Anspruch 1, **dadurch gekennzeichnet, dass** eine Zirkulation des Nebels aufrechterhalten wird, um die Erneuerung desselben zu erleichtern.

3. Verfahren nach Anspruch 1 oder 2, **dadurch gekennzeichnet, dass** das Dekontaminierungsprodukt Wasserstoffperoxid H₂O₂ ist.

4. Anlage zum Dekontaminieren der durchlaufenden Hälse (4a) von Vorformen (4), die nacheinander einer Aufgabevorrichtung (6) zugeführt werden, wobei die Vorformen (4) aus einem thermoplastischen Material und für die Herstellung von Behältern durch Blasen oder Ziehen/Blasen bestimmt sind, wobei die Dekontaminierungsanlage strukturell und funktionell mit einer Anlage (A) zur Zuführung der Vorformen verbunden ist, umfassend Mittel (4) zum Verschieben der Vorformen nacheinander, wobei die Dekontaminierungsanlage UV-Lampen (7) umfasst, die derart angeordnet sind, dass die ultraviolette Strahlung zur Gänze die Hälse (4a) der sich verschiebenden Vorformen (4) bestrahlen, **dadurch gekennzeichnet, dass** die Dekontaminierungsanlage ferner, stromaufwärts der den UV-Lampen (7) angeordnet ist, eine Kammer (10) umfasst, die von den Mitteln zum Verschieben der Vorformen der Zuführanlage (A) durchquert ist und in der Mittel (14) zum Zerstäuben eines Dekontaminierungsproduktes vorgesehen sind, die einen Nebel des Dekontaminierungsproduktes in der Kammer aufrechterhalten.

5. Anlage nach Anspruch 4, **dadurch gekennzeichnet, dass** die Zerstäubungsmittel (14) mindestens zwei Zerstäubungsdüsen (15) umfassen, die beiderseits der Mittel zum Verschieben der Vorformen und über diesen angeordnet sind, wobei ihre jeweiligen Achsen (19) im Wesentlichen in Richtung der Hälse (4a) der sich verschiebenden Vorformen (4) gerichtet sind.

6. Anlage nach Anspruch 4 oder 5, **dadurch gekennzeichnet, dass** Absaugmittel (20) an die Kammer (10) angeschlossen sind, um in dieser einen Zirkulationsstrom zu bilden, der lokale Anhäufungen des Produkts in der Schwebe verhindern kann.

7. Anlage nach einem der Ansprüche 4 bis 6, **dadurch gekennzeichnet, dass** eine Stange (23) im Inneren der Kammer (10) über den Mitteln zum Verschieben der Vorformen über den Hälsen (4a) der Vorformen mit relativ geringer Querabmessung in Bezug auf den Durchmesser der Hälse befestigt ist, wobei diese Stange ein Element darstellt, das das Anheben der Vorformen verhindert und gleichzeitig einen Zugang zum Nebel des Dekontaminierungsprodukts für die Innenwand der Hälse der Vorformen lässt.

8. Anlage nach einem der Ansprüche 4 bis 7, **dadurch gekennzeichnet, dass** die Mittel zum Verschieben der Vorformen eine geneigte Gleitschiene (5) umfassen, auf der die Vorformen (4) durch Schwerkraft hintereinander gleiten, und dass die Kammer (10) von dieser Gleitschiene (5) durchquert wird.
